# EUROPEAN PATENT SPECIFICATION

(11) **EP 0 628 073 B1**
(45) Date of publication and mention of the grant of the patent: **26.04.2000**
(21) Application number: 94902879.9
(22) Date of filing: 16.12.1993
(51) Int. Cl.: C12N 1/00, A23L 1/304, C02F 3/34, C02F 3/32, B01J 20/24

(54) **IONIC BINDING OF MICROBIAL BIOMASS**
IONENBINDUNG MIKROBIELLER BIOMASSE
LIAISON IONIQUE DE BIOMASSE MICROBIENNE

(30) Priority: 16.12.1992 GB 9226182
(43) Date of publication of application: 14.12.1994
(73) Proprietor: BIOTECHNA ENVIRONMENTAL INTERNATIONAL LIMITED, The Valley (AI)
(72) Inventor: HUGHES, Martin Neville, Kings College London, London W8 7AH (GB); POOLE, Robert Keith, Kings College London, London W8 7AH (GB); BENNETT, Sarah Melanie, Kings College London, London W8 7AH (GB)
(74) Representative: March, Gary Clifford
(86) International application number: GB9302568
(87) International publication number: WO9413782

(56) References cited:
- EP-A- 0 167 840
- EP-A- 0 181 497
- DE-A- 2 342 913
- DATABASE WPI Section Ch, Week 9313, Derwent Publications Ltd., London, GB; Class B06, AN 93-107670 & SU,A,1 726 371 (UNIV. MOLD) 15 April 1992
- CHEMICAL ABSTRACTS, vol. 110, no. 1, 2 January 1989, Columbus, Ohio, US; abstract no. 2418f, B. GREENE ET AL. 'TEMPERATURE DEPENDENCE OF METAL ION SORPTION BY SPIRULINA.' page 226 ; & BIORECOVERY vol. 1, no. 1 , 1988 pages 27 - 41

## Description

Certain microbial species have commercial application as a source of fine chemicals, lipids, polysaccharides and single cell protein. Microbial cell surfaces are negatively charged and therefore have a low affinity for anions, except at sites specifically designed for uptake of essential anions.

The cyanobacterium Spirulina platensis is well known in the health food market, being a non-toxic bacterium with intrinsic nutritional value. Its protein content is some 50-70% of dry-weight and its amino acid composition is well balanced. Its lipid content is of the order of 11% of dry weight whilst its vitamin B12 content is probably the highest of any unprocessed plant or animal food. Because of the lack of cellulose in the Spirulina cell wall, it is readily assimilated by humans. In some parts of the World, Spirulina is a natural foodstuff.

Research in the pharmaceutical and health food industries demonstrates that mineral deficiencies in the diet can be alleviated by ingesting minerals contained within, e.g. mineral tablets or in physical admixture or combination with a carrier foodstuff. In the case of soluble chemical compounds containing the required mineral, digestion or assimilation may be too rapid for the body to absorb beneficial quantities.

Two such metallic or metalloid minerals, which are important and have been implicated in mineral deficiency are zinc and selenium. These elements, at least in the form of ions in solution play an important role in metabolism and general good health. Whilst there is a need generally to ensure an adequate level of bioavailable zinc and selenium in the human diet, there have been problems in incorporating, for example, zinc and selenium minerals within supplements for foodstuffs.

Spirulina biomass, for example, contains low concentrations of metals taken up from the nutrient solution in which the cells were grown. Samples of Spirulina after combustion and ashing showed 10% metals of the total dry weight. Magnesium (in chlorophyll), potassium and sodium were generally always present.

Previous but unsuccessful attempts have been made to introduce metals into Spirulina on a commercial scale to supplement the amounts of metals taken up naturally.

The present inventors have now discovered that a range of nutritionally essential or desirable metal cations can be attached to the cell wall of microbial biomass and thereafter selected anions can be attached which either render the treated biomass suitable for the production of mineral supplements where the anionic species are also nutritionally essential or desirable or suitable for environmental clean up uses, where it is desired to remove toxic, contaminant or other hazardous or undesirable anions from an aqueous medium containing them.

According to one aspect of this invention there is provided microbial biomass cells wherein at least one metal cationic species is attached to the surface of said cells in an amount sufficient to render the said cells effective to bind anions thereto, and wherein at least one anionic species is also bound to the surface of said cells.

According to a second aspect of this invention there is provided biomass cells according to the said one aspect, in a form suitable for admixture into a digestible composition for consumption by humans or animals, or a digestible, mineral-containing composition which contains biomass cells according to the first aspect, or any preferred feature thereof.

According to a third aspect there is provided a method of making microbial biomass cells which comprises:
providing biomass cells within a culture medium, removing said cells from said medium and washing them,
suspending the cells in a buffer medium, adding a quantity of the required cationic species in aqueous solution optionally to saturation of the binding sites therefor on the cell wall,
adding a quantity of the required anionic species in aqueous solution optionally to saturation of the binding sites therefor on the cell wall,
removing the treated biomass and washing, and drying the cells to form a solid product.

According to a fourth aspect we provide a method of making a composition according to the second aspect, by mixing biomass cells as specified in the first aspect, in a dried state, with a biologically acceptable carrier or diluent, and converting the mixture to a form suitable for human or animal consumption.

According to a fifth aspect we provide a method of reducing the concentration of at least one anionic species in an aqueous composition containing said species, which comprises bringing said aqueous composition into intimate contact with microbial biomass cells, to which at least one metal cationic species has been bound in a quantity sufficient to render said biomass cells receptive to bind said at least one anionic species. The biomass may be as defined in the first aspect with the proviso that the said anionic species are absent until said aqueous composition has been brought into said intimate contact.

According to a sixth aspect we provide apparatus constructed and adapted to carry into effect a method as defined in the fifth aspect, which comprises at least one receptacle containing biomass affixed to an immobilised support, an inlet for receiving said aqueous composition and an outlet for the treated aqueous composition, wherein the biomass is as defined in any one of claims 1 to 7, with the proviso that the said anionic species are absent until an aqueous composition containing said anionic species to be treated has been brought into intimate contact with said biomass.

Such an apparatus may comprise two receptacles, arranged in series or in parallel. If two receptacles are used in parallel, it is possible to have a switching means to direct flow in flux from one to the other, whilst that receptacle not in use is regenerated. Regeneration of the biomass in such receptacles may be effected by acid washing to remove the bound anions and cations. Thereafter a flow of a cation containing solution e.g. zinc chloride or nitrate may be used to attach further zinc cations to the immobilised biomass.

It is preferred that the metal cations are divalent or trivalent such cations, preferably cations of essentially non-toxic transition metal elements, more preferably those which are essential or desirable minerals for the human or animal diet. For example we prefer the following cations: Zn(II), Cr(III), Cu(II), Mn(II) and to a lesser extent it may be possible to attach cations derived from cobalt, and magnesium as these can be useful trace minerals.

### Binding of metal cation to microbial surfaces

Metal cations are preferred which bind strongly to the cell surface. These include the later divalent transition metals (preferably essential elements) or more highly charged species. Good examples are zinc and chromium for digestible compositions. A metal cation can be added to cell surfaces by titrating into a suspension of the cells a solution of the required metal until free cations can be detected in solution. It is also possible to saturate the cell surface by binding several different metals sequentially if desired or even simultaneously. This can be done by titrating individual metals on to the cell surface to a required fraction of the total capacity of the cells for that metal ion. The amount of cation loaded onto cells can also be controlled by choice of pH conditions or, more straightforwardly, by choice of buffering agent. Any buffering species which contains a group that binds metal ions strongly will reduce the binding of that metal ion to cell walls. There may be several alternative types of binding site available on the cell surface with different preferences for metal cations.

In order that the skilled reader might select an appropriate biomass material and then bind metal cations of choice to the cell surface, a technique known as differential pulse polarography may be used to monitor the results and indicate quantitative binding capacities.

For example the reader is referred to a paper entitled Differential pulse polarography: a method of directly measuring uptake of metal ions by live bacteria without separation of biomass and medium - FEMS Microbiology Letters 92 (1992) pp 181-186.

The microbial species which are employed for nutritional purposes are preferably edible cyanobacteria, more preferably Spirulina. For environmental pollution clean-up purposes, the microbial species preferred for the biomass are preferably bacterial, more preferably cyanobacterial and most preferably spirulina and/or Bacillus.

### Binding of anions to metal cation treated biomass

When binding anions to the cation-laden cell surfaces there may be competition between binding of the required anion and the binding of any other anions present in the solution (usually the counter anion added with the metal ion). For maximum binding of the required anion we have found it preferable to prepare the cell surfaces using metal nitrate salts rather than chlorides, for example, as nitrate anions have been found to bind less strongly to metal centres than do chloride anions. However since the requirements for bioavailable selenium in digestible supplements compositions (such as mineral tablets or other food) is much lower than the requirement for zinc, it is preferred to use zinc chloride when binding the cell surfaces with zinc cations, as this can result subsequently in only partial occupancy of the total available cationic sites by selenium oxyanions.

The binding anions for use in the eventual preparation of digestible compositions containing the essential and/or desirable mineral elements are preferably metalloid and/or non-metal-oxyanions for example selenite SeO₃²⁻, selenate SeO₄²⁻, or borate BO₃³⁻. These can provide convenient dietary source of bioavailable selenium (or boron) especially in combination with or otherwise derived from biomass cells which can improve biological uptake.

In order that all aspects of the present invention may be more easily appreciated and readily carried into effect, preferred embodiments thereof are now described purely by way of non-limiting example only. Where explanations of mechanism have been given throughout this specification these are only for illustration and possible explanation only and are not to be considered as binding statements upon the applicants.

The invention in all its aspects is based on a novel approach to the problems of binding anionic species to the negatively charged surface of biomass for example, binding of selenium oxyanions to Spirulina to provide the mineral selenium in a bioavailable form. We have now surprisingly found methods for treating the cell walls of microbial biomass such as edible cyanobacteria, to vastly improve its capacity to take up this metalloid mineral element. The invention could be carried into effect with other types of biomass including yeasts, bacteria and different cyanobacteria.

In order to perform useful embodiments of the invention, the cell wall or surface of the edible bacteria Spirulina can be saturated with an aqueous solution containing metal cations such as Zn²⁺ or Cr³⁺. It has unexpectedly been found that when sufficient amounts of cations such as these have been bound to the cell surface, it then becomes feasible to subsequently bind anions e.g. metalloid - or non-metal oxyanions to the cell surface. Zinc ions have been found to bind relatively easily to the Spirulina surface and the available binding sites apparently can be saturated by treating e.g. Spirulina platensis with appropriate concentrations of the zinc ion by using an aqueous solution of a zinc salt. The determination of the uptake of zinc and the capacity for other metal cation uptake can be established using differential pulse polarography, as previously mentioned.

Additionally, it has also been found that addition of suitable, nutritionally desirable anions e.g. SeO₃²⁻, SeO₄²⁻, or BO₃²⁻ to the combined zinc ion Spirulina suspension, results in a synergistic further uptake of the zinc ion from the solution. The selenium oxyanion may be binding to the "bridged" zinc ions on the Spirulina surface, or be attached to other available binding sites on the cell surface which quite unexpectedly seems to synergise further uptake, i.e. binding to the cell surface of zinc ion.

The selenite ion, as an example of a potentially useful binding anion, appears to be taken up in quantities by Spirulina platensis which depend upon the pH of the aqueous solution containing such ions, and/or the nature of the buffer solution in which the biomass is maintained.

Digestible compositions such as foodstuff supplements or mineral tablets containing e.g. selenium and/or zinc together with one or more nutritionally essential or desirable mineral elements in the form of their corresponding anionic species are contemplated. For example within a biomass it is possible to bind selenium, zinc, manganese, chromium and boron in the form of their corresponding ionic species. When the required mineral elements have been attached to the Spirulina cell surface the cells can be filtered from suspension, washed, dried, and further processed into tablet or other supplement form. A particular advantage is that a high protein foodstuff can be prepared with precisely controlled amounts of suitable mineral elements, in a form which lends itself to suitable biological uptake.

In order to carry out the method under carefully controlled growing conditions for the biomass, it has been found particularly convenient to use an apparatus specially designed for the production of biomass manufactured and sold by Biotechna Limited under the BIOCOIL trademark. For a description and illustration of this coiled photobioreactor, the reader is referred to the description and drawings in EP-A-0239372.

### Environmental clean-up applications.

The present invention can also be used in methods of removing toxic anionic species such as selenate by 'biosorption' to the microbial cell surface. In some regions of the World there is excessive selenium or other toxic ionic species in water and so it is desirable to reduce its concentration. Accordingly the invention finds application not only in the production of digestible compositions which contain selenium as a nutritionally essential mineral, for human or animal consumption but also finds application in reducing the levels of principally anionic species normally associated with environmental water pollution. For example the invention can be used for removal of contaminant, possibly toxic anions from water containing them. The cation-treated biomass can thus be used, for example, as a cation-saturated scavenger bound to an immobilised support for removing unwanted, contaminant anions such as arsenate, chromate or chloro-complexes of precious metals from process streams and effluents and other metal-containing anionic contaminants from industrial processes. Moreover those aspects of the present invention which are concerned with environmental clean up of contaminate anionic species dissolved in water could be particularly attractive since the biomass cells, lend themselves to possible regeneration. For example when biomass cells on an immobilised support have become sufficiently loaded with or even saturated with the contaminant anionic species, the anions and cations could be removed with appropriate acid solutions and then the biomass could be re-activated by loading with suitable cationic species, e.g. zinc ions, for subsequent reuse.

The following procedure of Examples 1 to 4 was used to prepare zinc-laden samples of Spirulina platensis which could then be used to bind negatively-charged ionic species, i.e. the selected anions .

### Example 1

A 200 ml culture of Spirulina platensis was grown up in a standard medium. The cells were separated from the growth medium by centrifugation, washed with water and suspended in a HEPES buffer at pH 7.4. A 0.9 ml sample of this suspension was placed in a polarographic cell and a 50 mM solution of a zinc salt (either nitrate or chloride) was added with stirring until polarographic measurements indicated the presence of free zinc ions in solution. At this stage the binding sites for zinc on the Spirulina cell surfaces were saturated. The culture of Spirulina was then treated with a proportionate volume of the zinc solution, with stirring, so that the binding sites for zinc in the entire suspension would be fully saturated. The zinc content of the Spirulina was confirmed by acid digestion of a sample of the zinc-laden culture and direct measurement of the zinc by atomic absorption analysis. This measurement was not essential but served to confirm the accuracy of the polarographic titration. An aqueous solution of 50 mM sodium selenite or 50 mM sodium selenate (of equal volume to that of the 50 mM zinc solution) was then added to the suspension of zinc-laden Spirulina cells and allowed to stand with gentle stirring for a few minutes. The Spirulina was removed by centrifugation, the cells washed with water and the suspension freeze dried to give a solid product. The amount of selenium in the product depended upon the identity of the counter anion added with the zinc, as these compete with the selenium oxy anions for sites.

### Example 2

Example 1 was repeated on smaller and larger scales, each of which was repeated six times with consistent results.

### Example 3

The zinc-saturated Spirulina platensis and Bacillus subtilis have been treated with solutions containing selenate or selenite anions and the extent of uptake monitored by observing the decrease in concentration of that anion in solution. The effect of chloride ions on uptake of selenite by Spirulina platensis cells prepared using zinc chloride was also investigated. The product was treated with a solution of sodium selenite containing an equal number of moles to the surface bound zinc. It was found that the zinc-laden cells took up about 4 mg selenite per gramme of dry weight of cells, about twice the amount of selenite taken up by zinc-free cells, substantially less than anticipated. This fall in selenite uptake resulted from competition with the chloride. The selenite was bound to the zinc, as the zinc and the selenium could only be removed together, for example by repeated acid washing of the cells.

### Example 4

A variety of nutritionally essential metals has been added singly or in combination to the Spirulina cells by a similar procedure to that described above. The metal-laden cells were used to bind several anionic species, including borate and selenate ions.

### Example 5

Using a procedure similar to Example 1, samples were prepared from Spirulina platensis and Bacillus subtilis, a gram-positive organism with a high capacity for binding metal cations. In both cases we have bound a number of cation types, singly or in combination, to the cell surface to saturate the available binding sites on the biomass. We have bound singly or in combination, simultaneously and sequentially the cations of zinc(II), chromium(III), copper(II), manganese(II), cadmium(II) and lead(II) to cell surfaces, the latter two for illustration since these are undesirable for the human or animal diet. In applications other than environmental clean-up, it may not be necessary to saturate binding sites for cations.

For all these cations just described with the exception of chromium, the binding of the metal was straightforward and was usually maximum at around pH 6 to 8, with typical binding capacities around 30 mg per gramme of biomass, although this was dependent upon pH and buffer type. In the case of Spirulina platensis, polarographic techniques showed that at pH 7.4 a maximum of 310 mg of zinc per gramme dry weight of Spirulina can be added (for saturation of binding sites). This was confirmed by direct analysis of the cells by atomic absorption spectroscopy. Any amount of zinc may be added up to this limit. At pH 4.5, uptake was substantially lower at 18 mg per gramme of dry weight. In the case of Bacilus subtilis at pH 4.5 this value was reduced to 3.3 mg.

The binding of chromium(III) to cell surface was expected to occur slowly compared to the other cations. Polarographic analysis of solutions of hexaaquachromium(III) chloride showed the formation of a second species on standing. This was shown to be the first product of hydrolysis of the hexaaquachromium(III) cation, namely the hydroxo-bridged dimer. Continued polarographic monitoring of the solution in the presence of Spirulina platensis showed that the dimer was taken up rapidly onto the cell surface while the original species was taken up more slowly. The Cr(III) was bound most firmly of all the metals to the cells surfaces and could not be displaced by other transition metals or by dilute acid.

We have shown that lowering of the pH of the buffer in which the cyanobacterium Spirulina platensis is suspended results in higher binding affinities for selenium oxyanions (for example) as protonation of negative sites increases the possibility that negatively charged species will bind to the cell wall. However, anionic sites on cell surfaces are neutralised more effectively by binding metal cations rather than protons, as the former species will have higher positive charges, so allowing the generation of positively charged centres for binding anions.

## Claims

1. Isolated microbial biomass cells wherein cations of at least one metal cationic species are bonded to saturation to the surface of said cells rendering the said cells more effective to bind anions thereto, and wherein anions of at least one anionic species are also bonded to said biomass cells.

2. Microbial bacterial biomass cells wherein cations of at least one metal cationic species are bonded to saturation to the surface of said cells rendering the said cells more effective to bind anions thereto, and wherein anions of at least one anionic species are also bonded to said biomass cells.

3. Biomass cells as claimed in claim 1 or 2 wherein a plurality of different metal cationic species are attached to the said cells.

4. Biomass cells as claimed in claim 1 or 2 to which a plurality of different anionic species are also attached.

5. Biomass cells as claimed in any preceding claim which are derived from edible cyanobacteria.

6. Biomass cells as claimed in claim 5 derived from bacteria of the genus Spirulina or Bacillus.

7. Biomass cells as claimed in any preceding claim, wherein the cationic species comprises one or more of the following, namely the cations derived from: zinc, chromium, copper, manganese, cobalt and magnesium.

8. Biomass cells as claimed in any preceding claim wherein the anions comprise oxyanions of one or more metalloids and/or oxyanions of one or more non-metals.

9. Biomass cells as claimed in any preceding claim, in a form suitable for admixture into a digestible composition for consumption by humans or animals.

10. Biomass cells as claimed in claim 8 or 9 comprising zinc cations and one or more of the following oxyanions: selenate, selenite and borate.

11. A method of making microbial biomass cells as claimed in any preceding claims which comprises:
providing biomass cells within a culture medium, removing said cells from said medium and washing them,
suspending the cells in a buffer medium, adding a quantity of the required cationic species in aqueous solution to saturation of the binding sites therefor on the cell wall,
adding a quantity of the required anionic species in aqueous solution optionally to saturation of the binding sites therefor on the cell wall,
removing the treated biomass and washing, and drying the cells to form a solid product.

12. A method as claimed in claim 11 wherein cations of more than one cationic species are added simultaneously or sequentially.

13. A method as claimed in claim 11 or 12 wherein anions of more than one anionic species are added simultaneously or sequentially.

14. A digestible, mineral - containing composition which contains biomass cells as claimed in any one of claims 1 to 10.

15. A method of making a composition as claimed in claim 14, which comprises mixing biomass cells as claimed in any one of claims 1 to 10, in a dried state, with a biologically acceptable carrier or diluent, and converting the mixture to a form suitable for human or animal consumption.

16. A method of reducing the concentration of at least one anionic species in an aqueous composition containing said species, which comprises bringing said aqueous composition into intimate contact with microbial biomass cells, to which at least one metal cationic species has been bound in a quantity to saturation of the binding sites therefor on the cell wall, being sufficient to render said biomass cells receptive to bind said at least one anionic species.

17. A method as claimed in claim 16 which further involves causing the said biomass cells to become regenerated with the same or different metal cationic species after anionic species have become bound to said biomass.

18. A method as claimed in claim 16 or 17 wherein the biomass used comprises biomass as defined in any one of claims 1 to 10, with the proviso that the said anionic species are absent until said aqueous composition has been brought into said intimate contact.

## Patentansprüche

1. Isolierte mikrobielle Biomassezellen, bei denen Kationen wenigstens einer kationischen Metallart bis zur Sättigung an die Oberfläche der Zellen gebunden sind und dadurch die Zellen leistungsfähiger für die Bindung von Anionen gemacht sind, und bei denen Anionen wenigstens einer Anionenart ebenfalls an die genannten Biomassezellen gebunden sind.

2. Mikrobielle bakterielle Biomassezellen, bei denen Kationen wenigstens einer kationischen Metallart bis zur Sättigung an die Oberfläche der Zellen gebunden sind und dadurch die Zellen für die Bindung von Anionen leistungsfähiger gemacht sind, und bei denen Anionen wenigstens einer Anionenart ebenfalls an die Biomassezellen gebunden sind.

3. Biomassezellen nach Anspruch 1 oder 2, bei denen mehrere unterschiedliche kationische Metallarten an die genannten Zellen gebunden sind.

4. Biomassezellen nach Anspruch 1 oder 2, an die auch mehrere verschiedene Anionenarten gebunden sind.

5. Biomassezellen nach einem vorhergehenden Anspruch, die sich von essbaren Cyanobakterien ableiten.

6. Biomassezellen nach Anspruch 5, die sich von Bakterien der Art Spirulina oder Bacillus ableiten

7. Biomassezellen nach einem vorhergehenden Anspruch, bei denen die Kationenarten eine oder mehrere der folgenden umfassen, nämlich die Kationen, die sich von Zink, Chrom, Kupfer, Mangan, Kobalt und Magnesium ableiten.

8. Biomassezellen nach einem vorhergehenden Anspruch, bei denen die Anionen Oxyanionen eines oder mehrerer Metalloide und/oder Oxyanionen eines oder mehrerer Nichtmetalle umfassen.

9. Biomassezellen nach einem vorhergehenden Anspruch in einer geeigneten Form für die Beimischung zu einer verdaubaren Zusammensetzung für den Verbrauch durch Menschen oder Tiere.

10. Biomassezellen nach Anspruch 8 oder 9, mit Zink-Kationen und einem oder mehreren der folgenden Oxyanionen: Selenat, Selenit und Borat.

11. Verfahren zur Herstellung mikrobieller Biomassezellen nach einem vorhergehenden Anspruch, bei dem man
Biomassezellen in einem Nährmedium vorsieht, die Zellen aus dem Medium entfernt und wäscht,
die Zellen in einem Puffermedium suspendiert, eine Menge der erforderlichen Kationenart in wässriger Lösung bis zur Sättigung der Bindungsstellen für diese an der Zellwandung zusetzt,
eine Menge der erforderlichen Anionenart in wässriger Lösung wahlweise bis zur Sättigung der Bindungsstellen für diese an der Zellwandung zusetzt,
die behandelte Biomasse entfernt und wäscht und die Zellen unter Bildung eines festen Produkts trocknet.

12. Verfahren nach Anspruch 11, bei dem Kationen von mehr als einer Kationenart gleichzeitig oder nacheinander zugesetzt werden.

13. Verfahren nach Anspruch 12, bei dem Anionen von mehr als einer Anionenart gleichzeitig oder nacheinander zugesetzt werden.

14. Verdaubare, mineralhaltige Zusammensetzung, die Biomassezellen nach einem der Ansprüche 1 bis 10 enthält.

15. Verfahren zur Herstellung einer Zusammensetzung nach Anspruch 14, bei dem man Biomassezellen nach einem der Ansprüche 1 bis 10 in getrocknetem Zustand mit einem biologisch zulässigen Träger oder Verdünnungsmittel mischt und das Gemisch in eine für den menschlichen oder tierischen Verbrauch geeignete Form überführt.

16. Verfahren zur Verringerung der Konzentration wenigstens einer Anionenart in einer diese Art enthaltenden, wässrigen Zusammensetzung, bei dem man die wässrige Zusammensetzung in innigen Kontakt mit den mikrobiellen Biomassezellen bringt, an die wenigstens eine kationische Metallart in einer Menge bis zur Sättigung der Bindungsstellen für sie auf der Zellwand gebunden wurde, die ausreicht, um die Biomassezellen für die Bindung der wenigstens einen Anionenart aufnahmefähig zu machen.

17. Verfahren nach Anspruch 16, bei dem man die Biomassezellen mit der gleichen oder einer verschiedenen kationischen Metallart regeneriert, nachdem die Anionenarten an die Biomasse gebunden wurden.

18. Verfahren nach Anspruch 16 oder 17, bei dem die benutzte Biomasse eine in einem der Ansprüche 1 bis 10 definierte Biomasse umfaßt mit der Maßgabe, daß die genannten Anionenarten abwesend sind, bis die genannte wässrige Zusammensetzung in den genannten innigen Kontakt gebracht wurde.

## Revendications

1. Cellules de biomasse microbienne isolées, dans lesquelles des cations d'au moins une espèce cationique métallique sont liées jusqu'à saturation à la surface desdites cellules, rendant lesdites cellules plus efficaces pour y lier des anions, et dans lesquelles des anions d'au moins une espèce anionique sont également liés auxdites cellules de biomasse.

2. Cellules de biomasse bactérienne isolées, dans lesquelles des cations d'au moins une espèce cationique métallique sont liées jusqu'à saturation à la surface desdites cellules, rendant lesdites cellules plus efficaces pour y lier des anions, et dans lesquelles des anions d'au moins une espèce anionique sont également liés auxdites cellules de biomasse.

3. Cellules de biomasse selon la revendication 1 ou 2, dans lesquelles une pluralité d'espèces cationiques métalliques différentes sont fixées auxdites cellules.

4. Cellules de biomasse selon la revendication 1 ou 2, auxquelles sont également fixées une pluralité d'espèces anioniques différentes.

5. Cellules de biomasse selon l'une quelconque des revendications précédentes, qui sont dérivées de cyanobactéries comestibles.

6. Cellules de biomasse selon la revendication 5, dérivées de bactéries du genre *Spirulina* ou *Bacillus*.

7. Cellules de biomasse selon l'une quelconque des revendications précédentes, dans lesquelles les espèces cationiques comprennent un ou plusieurs des éléments suivants, à savoir les cations dérivés du zinc, du chrome, du cuivre, du manganèse, du cobalt et du magnésium.

8. Cellules de biomasse selon l'une quelconque des revendications précédentes, dans lesquelles les anions comprennent des oxyanions d'un ou plusieurs métalloïdes et/ou des oxyanions d'un ou plusieurs non-métaux.

9. Cellules de biomasse selon l'une quelconque des revendications précédentes, sous une forme adaptée pour être mélangées à une composition digestible en vue d'une consommation par des hommes ou des animaux.

10. Cellules de biomasse selon la revendication 8 ou 9, comprenant des cations zinc et un ou plusieurs des oxyanions suivants : sélénate, sélénite et borate.

11. Procédé de préparation de cellules de biomasse microbienne selon l'une quelconque des revendications précédentes, qui comprend les étapes consistant à :
disposer les cellules de biomasse dans un milieu de culture, retirer lesdites cellules dudit milieu et les laver,
mettre les cellules en suspension dans un milieu tampon, ajouter une certaine quantité de l'espèce cationique souhaitée en solution aqueuse jusqu'à saturation des sites de liaison prévus pour celle-ci sur la paroi cellulaire,
ajouter une certaine quantité de l'espèce anionique souhaitée en solution aqueuse éventuellement jusqu'à saturation des sites de liaison prévus pour celle-ci sur la paroi cellulaire,
retirer la biomasse traitée et laver et sécher les cellules pour former un produit solide.

12. Procédé selon la revendication 11, dans lequel des cations de plus d'une espèce cationique sont ajoutés simultanément ou les uns à la suite des autres.

13. Procédé selon la revendication 11 ou 12, dans lequel des anions de plus d'une espèce anionique sont ajoutés simultanément ou les uns à la suite des autres.

14. Composition digestible à base de minéraux qui contient des cellules de biomasse, selon l'une quelconque des revendications 1 à 10.

15. Procédé de préparation d'une composition selon la revendication 14, qui comprend les étapes consistant à mélanger des cellules de biomasse selon l'une quelconque des revendications 1 à 10, à l'état séché, à un support ou un diluant biologiquement acceptable, et à convertir le mélange en une forme adaptée pour la consommation humaine ou animale.

16. Procédé de réduction de la concentration en au moins une espèce anionique dans une composition aqueuse contenant ladite espèce, qui comprend les étapes consistant à amener ladite composition aqueuse en contact intime avec les cellules de biomasse microbienne, auxquelles au moins une espèce cationique métallique a été liée en une certaine quantité jusqu'à saturation des sites de liaison prévus pour celle-ci sur la paroi cellulaire, ce qui est suffisant pour rendre lesdites cellules de biomasse réceptives pour lier ladite au moins une espèce anionique.

17. Procédé selon la revendication 16, qui implique en outre le fait d'amener lesdites cellules de biomasse à se régénérer avec une espèce cationique métallique identique ou différente une fois que les espèces anioniques ont été liées à ladite biomasse.

18. Procédé selon la revendication 16 ou 17, dans lequel la biomasse utilisée comprend la biomasse selon l'une quelconque des revendications 1 à 10, à condition que ladite espèce anionique soit absente jusqu'à ce que ladite composition aqueuse ait été amenée en ledit contact intime.
